# EUROPEAN PATENT APPLICATION

(11) **EP 1 271 147 A1**
(43) Date of publication of application: **02.01.2003**
(21) Application number: 01912402.3
(22) Date of filing: 14.03.2001
(51) Int. Cl.: G01N 33/52, G01N 31/22

(54) **TEST PAPER**

(30) Priority: 17.03.2000 JP 2000081670
(71) Applicant: Sanwa Kagaku Kenkyusho Co., Ltd., Nagoya-shi, Aichi 461-8631 (JP)
(72) Inventor: Komagoe, Yoshiyuki, Sanwa Kagaku Kenkyusho Co. Ltd, Nagoya-shi, Aichi 461-8631 (JP); Takehiro, Osamu, Sanwa Kagaku Kenkyusho Co., Ltd., Nagoya-shi, Aichi 461-8631 (JP); Asai, Noboru, Sanwa Kagaku Kenkyusho Co., Ltd., Nagoya-shi, Aichi461-8631 (JP)
(74) Representative: Lippert, Hans, Dipl.-Ing.
(86) International application number: JP0102064
(87) International publication number: WO01069246

(57) **Abstract**

It is directed to achieve quantitative measurement over a wide concentration range and good repeatability in a test paper for analyzing and measuring the color of a substance to be analyzed with a color developing reagent.

A test paper is produced in which the development layer and the reagent layer are separated from each other and the coloring in the reagent layer is migrated on the development layer for measurement. In addition, a specimen-absorbing layer is provided next to the development layer, thereby further improving the flow of specimens. A sample addition section contains an agglutinating agent that has the function of blood cell separation or/and separation membrane is provided between the reagent layer and the development layer, particularly when whole blood samples are used.

## Description

### Technical Field

The present invention relates to a test paper used to analyze and measure, quantitatively or qualitatively, given components in a liquid sample, based on development of color in the field of clinical chemistry or others.

### Background Art

Conventionally, in test papers that involve the development of color during a chemical reaction, a single-layered test paper having a filter paper, or an equivalent thereof, impregnated with a reagent is used and samples are dropped on the same layer thereof to develop a color which is measured with the naked eyes or by using a simple reflectance meter, as described in, for example, United States Patent Nos. 3,050,373 and 3,061,523.

As test papers having a multilayer structure, a test paper comprising a reagent layer and a developing layer is disclosed in, for example, Japanese Patent Laid-Open No.H04-304899 and Japanese Patent Publication No.H06-104077. The developing layers are a sample addition section located upstream of the reagent layer. The reagent layer is used as the place where the development of color and determination are made. In addition, Japanese Patent Laid-Open No.H09-184837 discloses a test piece that comprises an independent detection layer. The independent detection layer allows pigments, after the development of their color, to migrate in the direction perpendicular to the reagent layer for adsorption and determination in order to improve detection in a low concentration range. This provides good measurement sensitivity in the low concentration range. On the contrary, development of color of the pigments is leveled off in a high concentration ranges. Accordingly, it may become necessary that measurement be made again when the sample contains an unexpectedly large amount of substance to be analyzed. It is thus insufficient from a viewpoint of the quantitative measurement.

Furthermore, in addition to problems from the viewpoint of the quantitative measurement, these prior arts are inferior in measurement repeatability due to a bias of the pigments after the development of their color.

On the other hand, in order to detect a certain component in a blood sample that is not subjected to separation of solid components such as red blood cells (hereinafter, referred to as a "whole blood sample"), a pre-treatment step is necessary to remove the solid components such as the red blood cells from the whole blood sample before detection of the substance to be analyzed with a test paper. Japanese Patent Laid-Open No.S57-53661 discloses blood analysis using a layer of glass fibers while Japanese Patent Laid-Open No.S63-177059 discloses a device for separating plasma using soluble agglutinins in combination with a filter, as such pre-treatment step or an analytical process that involves the pre-treatment step.

Conventional test papers that rely on color reactions have problems such as a narrow concentration range available for quantitative measurement and inferior measurement repeatability. In addition to the above-mentioned problems, measurement is difficult for whole blood samples when the amount of the whole blood samples is small because a blood cell separation layer is placed in front of a reagent layer.

The present invention was made with respect to the above-mentioned problems. Thus, an object thereof is to improve measurement repeatability and provide a test paper with which a substance to be analyzed can be detected quantitatively over a wide range, from low to high concentrations, and a substance to be analyzed can be measured even in a small amount of a whole blood sample.

### Summary of the Invention

The present inventors have focused on the fact that carriers in test papers that rely on color reactions are required to have different properties for the addition of a sample, maintenance of color developing reagent, color reaction and measurement of development of color. The present inventors have made it possible to expand the concentration range available for the quantitative measurement and improve the measurement repeatability by means of providing a development layer as another layer separated from a reagent layer, and using optimum carriers for each of these to diffuse and develop a color developing substance, whose color has already been developed in a reagent layer, over a development layer for identification. Furthermore, some cases use a configuration in which a sample addition layer is separated from the reagent layer or a configuration in which a specimen-absorbing layer is provided next to the development layer.

With whole blood samples, in addition to the above-mentioned configurations, a red blood cell filtering function is provided by means of, for example, combining a reagent layer and/or a sample addition layer with an agglutinating agent for red blood cells, and/or placing a plasma separation membrane between the reagent layer and development layer.

This red blood cell filtering function allow more thorough plasma separation, simultaneous separation and measurement of the plasma, enlargement of the concentration range available for the quantitative measurement and improvement of the measurement repeatability.

By using the configuration of the present invention for a test paper in which the color of a substance to be analyzed is developed and measured with a color developing reagent, it is possible to achieve quantitative measurement over a wide concentration range from low to high concentrations and good repeatability. In particular, when a whole blood sample is used, separation of red blood cells is conducted simultaneously with measurement thereof thereby allowing quantitative measurement over a wide concentration range and good repeatability, as well as measurement of a small amount of whole blood samples.

### Brief Description of the Drawings

FIG. 1 is an exploded perspective view of a three-layered test paper wherein a sample addition layer, a reagent layer, and a development layer are adhered on a substrate with certain overlapped portions;
FIG. 2 is a plane view (A) and a side view (B) of a three-layered test paper wherein a sample addition layer, a reagent layer, and a development layer are adhered on a substrate with certain overlapped portions;
FIG. 3 is an exploded perspective view of a two-layered test paper wherein a reagent layer having a sample addition section and a development layer are adhered on a substrate with a certain overlapped portion;
FIG. 4 is a plane view (A) and a side view (B) of a two-layered test paper wherein a reagent layer having a sample addition section and a development layer are adhered on a substrate with a certain overlapped portion;
FIG. 5 is an exploded perspective view of a four-layered test paper wherein a reagent layer having a sample addition section, a plasma separation membrane, a development layer, and a specimen-absorbing layer are adhered on a substrate with certain overlapped portions;
FIG. 6 is a plane view (A) and a side view (B) of a four-layered test paper wherein a reagent layer having a sample addition section, a plasma separation membrane, a development layer, and a specimen-absorbing layer are adhered on a substrate with certain overlapped portions;
FIG. 7 is a calibration curve in which the abscissa represents the concentration of a 3-hydroxybutyrate solution while the ordinate represents the reciprocal number of reflection rate. The reciprocal number of the reflection rate is in proportion to the color development concentration. The quantitative measurement range is the range where a linearity is observed between the color development concentration (the reciprocal number of the reflection rate) and the concentration of the 3-hydroxybutyrate solution;
FIG. 8 is a calibration curve in which the abscissa represents the concentration of a glucose solution while the ordinate represents the reciprocal number of the reflection rate. The reciprocal number of the reflection rate is in proportion to the color development concentration. A quantitative measurement range is the range where a linearity is observed between the color development concentration (the reciprocal number of the reflection rate) and the concentration of the glucose solution;
FIG. 9 is a calibration curve in which the abscissa represents the concentration of whole blood supplemented with 3-hydroxybutyrate while the ordinate represents the reciprocal number of the reflection rate. The reciprocal number of the reflection rate is in proportion to the color development concentration. A quantitative measurement range is the range where a linearity is observed between the color development concentration (the reciprocal number of the reflection rate) and the concentration of the 3-hydroxybutyrate; and
FIG. 10 is a calibration curve in which the ordinate represents the concentration of whole blood supplemented with 3-hydroxybutyrate while the abscissa represents the K/S value. The K/S value is obtained from the reflection rate (R) using the Kubelka-Munk equation, i.e., K/S = (1-R)²/2R. From the K/S value, the concentration of 3-hydroxybutyrate can be obtained using an approximate expression, Y = -514.06X⁵ + 2722.7X⁴ -4189.2X³ + 2322.7X² + 1375.8X - 27.357.

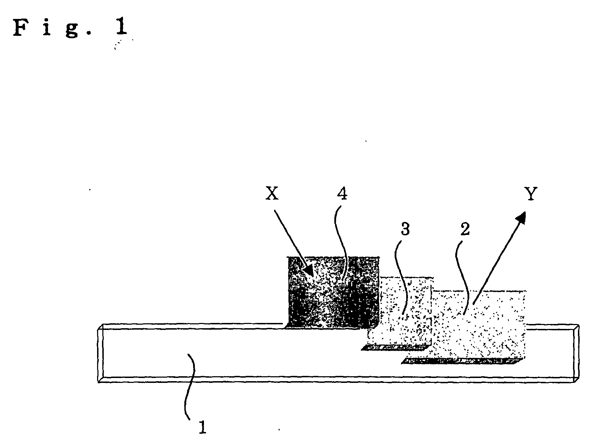

The following reference numerals are used in the figures. 1: substrate, 2: development layer, 3: reagent layer, 4: sample addition layer, 5: reagent layer having a sample addition section, 6: double-sided tape , 7: plasma separation membrane, 8: specimen-absorbing layer, 9: top laminate, X: addition of a liquid sample, and Y: measurement of the reflection rate.

### Best Mode for Carrying Out the Invention

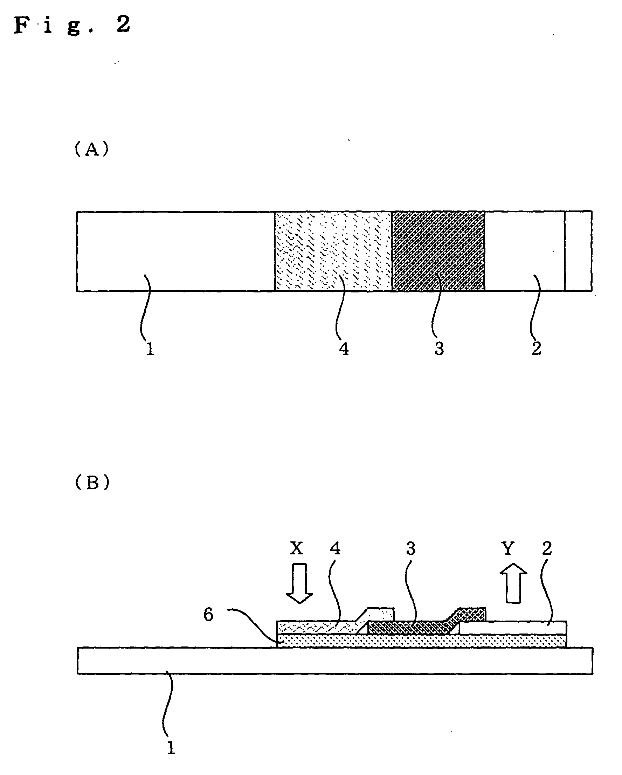

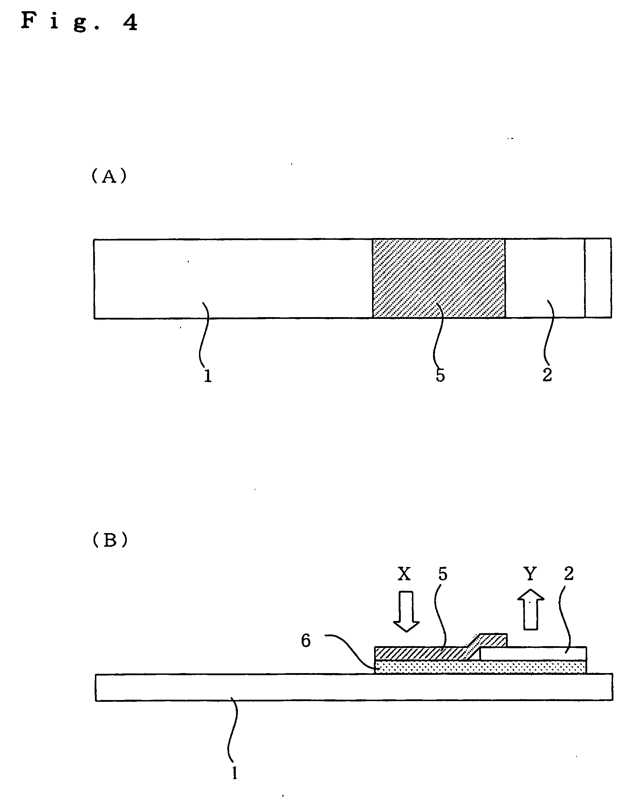

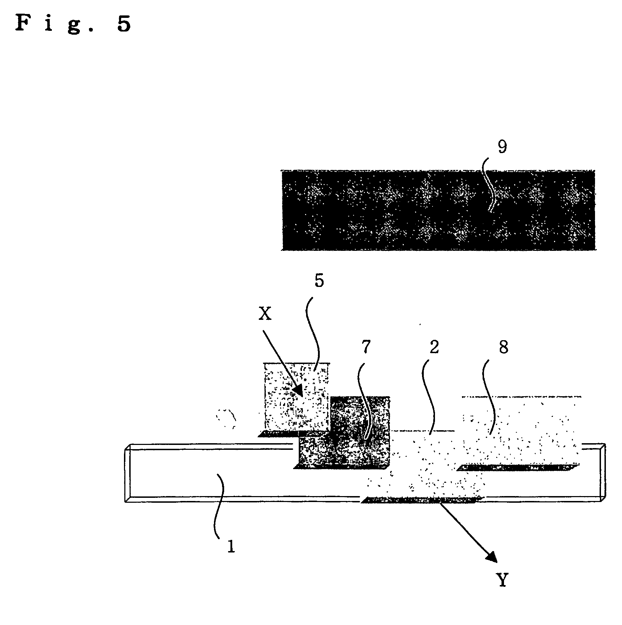

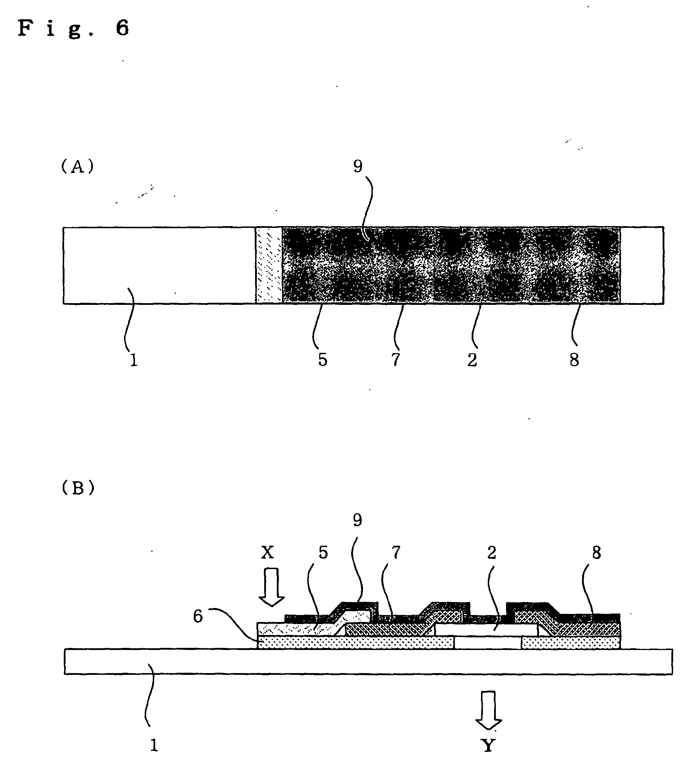

Test papers of the present invention are basically those comprising, as shown in FIGS. 1 to 6, a development layer 2 which is separated from reagent layer 3 or 5. The development layer 2 is the section where the color developing substance whose color has developed in the reagent layer 3 or 5 is diffused and developed for identification. It is preferable to provide a test paper in which a sample addition layer 4 is not separated from the reagent layer 3 (i.e., the reagent layer 5 having a sample addition section) in order to deal with small amounts of whole blood samples. On the other hand, it is typically preferable to provide a test paper comprising the sample addition layer 4 separated from the reagent layer 3 as shown in FIGS. 1 and 2 when, for example, a pre-treatment is required to remove impurities in a sample or the amount of samples is large. In addition, as shown in FIGS. 5 and 6, it is possible to improve the flow of specimens by providing a specimen-absorbing layer 8 next to the development layer 2. Furthermore, it is effective to incorporate a plasma separation membrane 7 between the reagent layer 5 having the sample addition section and the development layer 2 as shown in FIGS. 5 and 6 in order to achieve higher separability of the red blood cells. Of course, according to the present invention, the plasma separation membrane 7 may be incorporated between the reagent layer 3 and the development layer 2.

These layers are configured in a continuous form so that samples can migrate throughout by means of overlapping or contacting certain portions of the layers with each other. In addition, it is preferable that the layers are adhered on a water-proof substrate 1.

As to materials for the above-mentioned layers, glass fibers, cellulose, polypropylene, or filter papers may be used for the sample addition layer 4 and the reagent layer 3 or 5. Preferably, the layers may be formed of glass fibers having a thickness of 0.2 mm to 2.0 mm, and a density of 0.1 g/cm³ to 0.5 g/cm³. In addition, for the development layer 2, nitrocellulose, nylon, or poly vinylidene difluoride may be used, of which nitrocellulose is preferable. Here, it is preferable that the nitrocellulose used has, as properties thereof, a thickness of 100 µm to 200 µm and a flow rate of 10 sec/cm to 80 sec/cm. For the plasma separation membrane 7, commercially available plasma separation membranes may be used that are made of, for example, polyester or polyethersulfone. Glass fibers, cellulose, or filter papers may be used for the specimen-absorbing layer 8. For the water-proof substrate 1, plastics (e.g., polyethylene terephthalate, polyethylene, vinyl chloride, acrylics, ABS resins, styrol resins, and polycarbonate), glass or metals may be used, of which plastics are preferable. It is preferable that the water-proof substrate 1 is transparent in consideration of measurement of reflected light from the backside of the test paper or measurement with transmitted light.

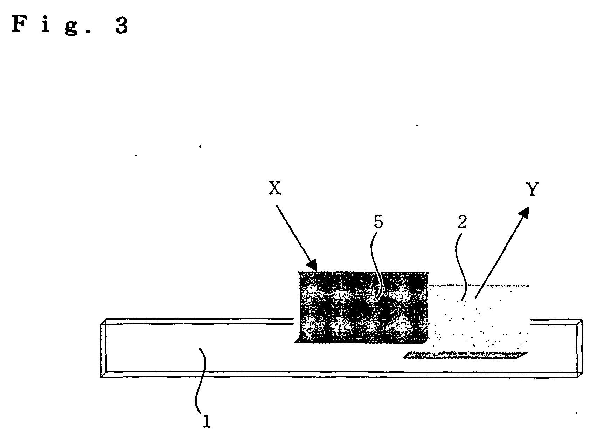

In the test paper of the present invention, the reagent that is necessary for the development of color is held in a dry form by the reagent layer 3 or 5. With the test paper having the sample addition layer 4 (shown in FIG. 1 or 2), liquid samples added to the sample addition layer 4 flow through the sample addition layer 4, the reagent layer 3, and the development layer 2 in this order. In addition, liquid samples added to the reagent layer 5 having the sample addition section (shown in FIGS. 3 to 6) flow through the reagent layer 5 and the development layer 2 in this order. In other words, the substance to be analyzed in the sample that has migrated or been added to the reagent layer 3 or 5 reacts with the reagent contained in the reagent layer 3 or 5 to form a color developing substance. Said color developing substance is diffused and developed over the development layer 2.

### Measurement or identification is made on the development layer 2.

The development layer 2 has the following effects among others. (1) By dispersing the pigments whose color has been developed in the reagent layer 3 or 5 into the development layer 2, a leveling off of the color development can be avoided for samples of high concentration and the range of determination can be expanded in the high concentration range. (2) The development layer 2 is not required to have a reagent holding capacity. Thus, a smooth and homogeneous material can be selected that is optimum for the coloring measurement. In addition, unevenness or non-uniform development of color is uniformized when the pigments are dispersed. This allows quantitative measurement with a good repeatability. (3) Choices of material (s) and sizes control the dispersion of the pigments and adjust the density of the developed color. The range of quantitative measurement can be varied to both a low concentration side and a high concentration side, depending on necessity. (4) In particular, when whole blood samples are used, the whole blood itself is subjected to reaction in the reagent layers. Separation of plasma can be performed after the completion of the color reaction. The necessary amount of whole blood specimens is an amount corresponding to a bed volume of the reagent layers. The only requirement is to provide plasma that fills a development layer having a small bed volume. It is possible to reduce the amount of the whole blood specimens to be added accordingly. These involve essential effects associated with the present invention as well as additional effects thereof.

A specimen-absorbing layer 8 may be provided next to the development layer 2. The specimen-absorbing layer 8 has the following effects among others. (1) The flow of the specimen continues after specimen saturation of the development layer 2, which is effective in providing uniform development of color. (2) By adjusting the balance of the specimen amount and the volume of the specimen-absorbing layer 8, the flow of the specimen can be blocked at any time. (3) When an excessive amount of specimen is added, overflow of the specimen from the test paper can be avoided.

Colors may be developed using, for example, a method in which a substance to be analyzed in a sample directly oxidizes or reduces pigment precursors to form a pigment or, alternatively, a method in which a substance to be analyzed in a sample reduces an oxidized coenzyme in the presence of enzyme , and the reduced coenzyme reduces a pigment precursor through an electron relay to reduce the pigment precursor, thereby forming a pigment.

Measurement or determination may be made by using, for example, a method in which change in color tone or/and concentration of the development layer 2 is/are determined with the naked eye, a method in which change in color tone or/and concentration is/are measured using reflected light with a reflection rate measurement device or using transmitted light with an absorbance meter to thereby measure the concentration of a substance to be analyzed in a sample.

For example, procedures with 3-hydroxybutyrate dehydrogenase may be used when the substance to be analyzed is blood 3-hydroxybutyrate. In such a case, as the reagent in the reagent layer 3 or 5, 3-hydroxybutyrate dehydrogenase, nicotinamide adenine dinucleotide (NAD⁺), diaphorase, and nitrotetrazolium blue are used. The 3-hydroxybutyrate in the sample is converted into acetoacetic acid in the reagent layer 3 or 5 while NAD⁺ is reduced into NADH. The NADH is used to develop the color of nitrotetrazolium blue with diaphorase. The purple color that appears on the development layer 2 reflects the amount of 3-hydroxybutyrate.

Procedures with glucose oxidase/peroxidase may be used when the substance to be analyzed is blood glucose. In such a case, glucose oxidase, peroxidase, aminoanitipirine, and phenol are used as the reagent in the reagent layer 3 or 5. The glucose in the sample is converted into glucono lactone in the reagent layer 3 or 5 and produces hydrogen peroxide. The hydrogen peroxide, aminoanitipirine, and phenol produce quinone pigments. The reddish color that appears on the development layer 2 reflects the amount of the glucose.

In the measurement of blood glucose level using a procedure with glucose dehydrogenase, as the reagent in the reagent layer 3 or 5, glucose dehydrogenase, NAD⁺, diaphorase, and nitrotetrazolium blue are used. Glucose in the sample is converted into glucono lactone in the reagent layer 3 or 5 while NAD⁺ is reduced into NADH. The NADH is used to develop the color of nitrotetrazolium blue with diaphorase. The purple color that appears on the development layer 2 reflects the amount of the glucose.

Any migration of red blood cells to the development layer 2 should be prevented particularly when whole blood samples are used. To this end, the reagent layer 3 or 5 or/and the sample addition layer 4 is imparted with a function to hold the red blood cells and filter plasma or/and a plasma separation membrane 7 is provided between the reagent layer 3 or 5 and the development layer 2 to separate the red blood cells.

For the former case, it is preferable to use an agglutinating agent capable of agglutinating red blood cells, such as lectin, an anti-erythrocyte antibody or a cationic polymer, and contain it in a carrier with a filtering capacity. The carrier having a filtering function is preferably a depth filter such as a glass fiber rather than a screen filter. A screen filter captures particles on the surface thereof and has a fixed pore diameter while a depth filter captures particles on the surface of and inside a fibrous structure and is thus less likely to become clogged. With glass fiber, a specific pore size cannot be defined because of the random structure thereof. However, it is preferable to use a glass filter having a thickness of 0.2 mm to 2.0 mm and a density of 0.1 g/cm³ to 0.5 g/cm³ with a capability of holding particles having a diameter of 1 µm or larger. The reason why a certain agglutinating agent is used is that glass fiber typically has a particle holding capacity of 1 µm or larger in diameter and red blood cells have a diameter of about 7 µm. However, the holding rate for particles having a diameter of 1 µm or larger is not 100%, and red blood cells are deformable.

The agglutinating agent may be contained by means of, for example, a filter impregnated with an aqueous solution of the agglutinating agent and then dried.

While the latter plasma separation membrane is already described, it may be used alone or be combined with the former filtering function. In particular when it is used in combination with the former filtering means containing an agglutinating agent, clogging of the plasma separation membrane is prevented through the effect of the agglutinating agent. A larger amount of plasma is separated as compared with cases where it is used alone, which is preferable.

### Examples

### Example 1

A glass fiber sample addition layer of 5 mm x 8 mm, a glass fiber reagent layer of 5 mm x 5 mm, and a nitrocellulose development layer of 5 mm x 10 mm were adhered in this order on a white polyethylene terephthalate substrate of 5 mm x 65 mm, with overlapped margins of 2 mm each to prepare test papers. The reagent layer had previously had 100 µl/cm² (25 µl/test) of a solution of 10 mg/ml of nicotinamide adenine dinucleotide, 50 units/ml of 3-hydroxybutyrate dehydrogenase, 200 units/ml of diaphorase, 10 mg/ml of bovine serum albumin, and 4.5 mg/ml of nitrotetrazolium blue in 40 mM phosphate buffer, pH 8.0 added and was dried under vacuum.
Glass Fiber (Sample Addition Layer): available from Millipore Corporation (USA) Cat. No. AP2029325
Glass Fiber (Reagent Layer): available from Millipore Corporation (USA) Cat. No. SA3J763H8
Nitrocellulose (Development Layer): available from Millipore Corporation (USA) Cat. No. STHF04000

### Comparative Example 1

A single layer of a filter paper of 5 mm x 8.3 mm was adhered on a white polyethylene terephthalate substrate of 5 mm x 65 mm to prepare test papers. As in the Example 1, the single layer had previously had 60 µl/cm² (25 µl/test) of a solution of 10 mg/ml of nicotinamide adenine dinucleotide, 50 units/ml of 3-hydroxybutyrate dehydrogenase, 200 units/ml of diaphorase, 10 mg/ml of bovine serum albumin, and 4.5 mg/ml of nitrotetrazolium blue in 40 mM phosphate buffer, pH 8.0 added and was dried under vacuum.
Filter Paper: available from Whatman (GB) Cat. No. 3001902

### Test Example 1

Added to the test papers according to the present invention obtained in the above-mentioned Example 1 and the test papers obtained in the Comparative Example 1 was 25 µl of 3-hydroxybutyrate solution in 1000 µM concentration. A reflection rate of 530 nm was measured 2 minutes later using a reflection rate measurement device. The results thereof are given in Table 1. As apparent from Table 1, the test papers according to the present invention exhibit less variation in development of color than the comparative example test papers.

**Table 1**

| 3-hydroxybutyrate simultaneous repeatability test | | | | | | | |
|---|---|---|---|---|---|---|---|
| | Reflection Rate(%) | | | | | | |
| | 1 | 2 | 3 | 4 | 5 | average | CV% |
| Test Papers of the Present Invention | 9.97 | 10.59 | 9.99 | 11.17 | 11.31 | 10.61 | 5.96% |
| Comparative Example Test Papers | 11.09 | 14.39 | 12.08 | 12.15 | 11.19 | 12.34 | 9.92% |

### Test Example 2

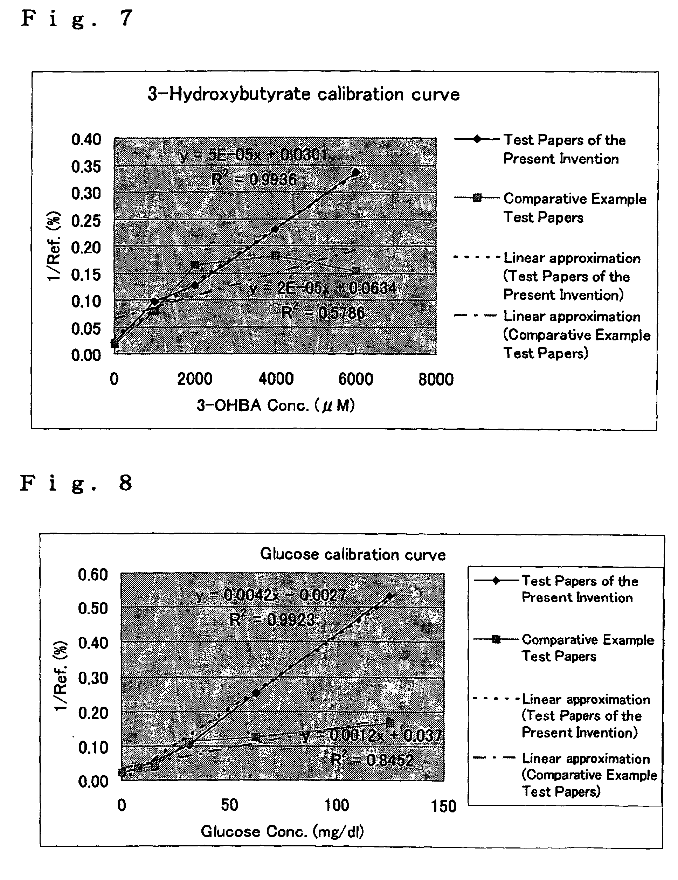

Added to the test papers according to the present invention (Example 1) and the comparative example test papers (Comparative Example 1) was 25 µl of 3-hydroxybutyrate solution in 0, 1000, 2000, 4000, and 6000 µM concentrations. A reflection rate of 530 nm was measured 2 minutes later using a reflection rate measurement device. A calibration curve was prepared with the abscissa representing the concentration of a 3-hydroxybutyrate solution and the ordinate representing the reciprocal number of the reflection rate. The results thereof are given in FIG. 7 and Table 2.

**Table 2**

| 3-hydroxybutyrate calibration curve | | | | | |
|---|---|---|---|---|---|
| | Reciprocal Number of Reflection Rate(%) | | | | |
| | 0 µM | 1000 µM | 2000 µM | 4000 µM | 6000 µM |
| Test Papers of the Present Invention | 0.0209 | 0.0973 | 0.1267 | 0.2304 | 0.3361 |
| Comparative Example Test Papers | 0.0198 | 0.0785 | 0.1639 | 0.1826 | 0.1533 |

As apparent from FIG. 7, the test papers of the present invention provide a wide concentration range in which the calibration curve has a linearity. The correlation coefficient of the calibration curve is R² = 0.9936 in the range of 0 µM to 6000 µM, which is better than the correlation coefficient of R² = 0.5786 obtained with the comparative example test papers.

The measurement range in which quantitative measurement can be made is 0 to 2000 µM for the comparative example test papers while the range can be enlarged to be 0 to 6000 µM or larger for the test papers of the present invention.

### Example 2

A glass fiber reagent layer having a sample addition section of 5 mm x 10 mm, and a nitrocellulose development layer of 5 mm x 10 mm were adhered in this order on a white polyethylene terephthalate substrate of 5 mm x 65 mm, with overlapped margins of 2 mm each to prepare test papers. The reagent layer had previously had 100 µl/cm² (50 µl/test) of a solution of 10 mg/ml of nicotinamide adenine dinucleotide, 50 units/ml of glucose dehydrogenase, 200 units/ml of diaphorase, 10 mg/ml of bovine serum albumin, and 4.5 mg/ml of nitrotetrazolium blue in 40 mM phosphate buffer, pH 8.0 added and was dried under vacuum.
Glass Fiber (Reagent Layer): available from Millipore Corporation (USA) Cat. No. SA3J763H8
Nitrocellulose (Development Layer): available from Millipore Corporation (USA) Cat. No. STHF04000

### Comparative Example 2

A single layer of a filter paper of 5 mm x 16.7 mm was adhered on a white polyethylene terephthalate substrate of 5 mm x 65 mm to prepare test papers. As in the Example 2, the single layer had previously had 60 µl/cm² (50 µl/test) of a solution of 10 mg/ml of nicotinamide adenine dinucleotide, 50 units/ml of glucose dehydrogenase, 200 units/ml of diaphorase, 10 mg/ml of bovine serum albumin, and 4.5 mg/ml of nitrotetrazolium blue in 40 mM phosphate buffer, pH 8.0 added and was dried under vacuum.
Filter Paper: available from Whatman (GB) Cat. No. 3001902

### Test Example 3

Added to the test papers according to the present invention obtained in the above-mentioned Example 2 and the test papers obtained in the Comparative Example 2 was 30 µl of glucose solution in 31 mg/dl concentration. A reflection rate of 530 nm was measured 2 minutes later using a reflection rate measurement device. The results thereof are given in Table 2. As apparent from Table 2, the test papers according to the present invention exhibit less variation in development of color than the comparative example test papers.

**Table 3**

| glucose simultaneous repeatability test | | | | | | | |
|---|---|---|---|---|---|---|---|
| | reflection rate(%) | | | | | | |
| | 1 | 2 | 3 | 4 | 5 | average | CV% |
| Test Papers of the Present Invention | 9.82 | 8.97 | 10.25 | 9.42 | 8.77 | 9.44 | 6.35% |
| Comparative Example Test Papers | 8.80 | 8.76 | 13.47 | 11.84 | 10.70 | 10.71 | 18.87% |

### Test Example 4

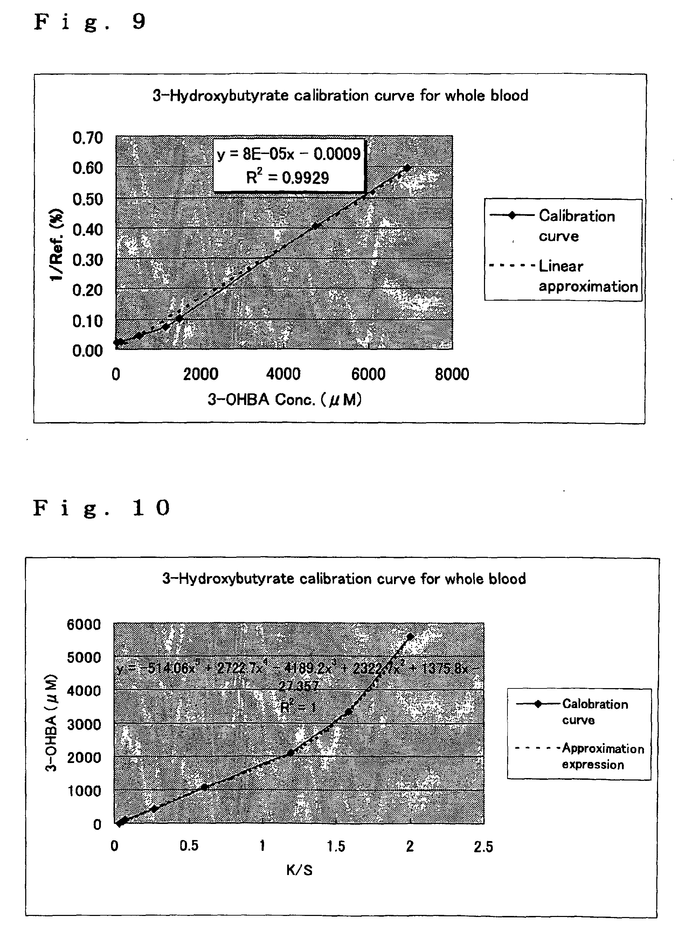

Added to the test papers according to the present invention (Example 2) and the comparative example test papers (Comparative Example 2) was 30 µl of glucose solution in 0, 7.8, 15.6, 31.25, 62.5, and 125 mg/dl concentrations. A reflection rate of 530 nm was measured 2 minutes later using a reflection rate measurement device. A calibration curve was prepared with the abscissa representing the concentration of a glucose solution and the ordinate representing the reciprocal number of the reflection rate. The results thereof are given in FIG. 8 and Table 4.

**Table 4**

| glucose calibration curve | | | | | | |
|---|---|---|---|---|---|---|
| | Reciprocal Number of Reflection Rate(%) | | | | | |
| | 0 mg/dl | 7.8 mg/dl | 15.6 mg/dl | 31.25 mg/dl | 62.25 mg/dl | 125 mg/dl |
| Test Papers of the Present Invention | 0.0195 | 0.0378 | 0.0573 | 0.1018 | 0.2519 | 0.5348 |
| Comparative Example Test Papers | 0.0219 | 0.0345 | 0.0409 | 0.1136 | 0.1242 | 0.1656 |

As apparent from FIG. 8, the test papers of the present invention provide a wide concentration range in which the calibration curve has a linearity. The correlation coefficient of the calibration curve is R² = 0.9923 in the range of 0 mg/dl to 125 mg/dl, which is better than the correlation coefficient of R² = 0.8452 obtained with the comparative test papers.

The measurement range in which quantitative measurement can be made is 0 to 31.25 mg/dl for the comparative example test papers while the range can be enlarged to be 0 to 125 mg/dl or larger for the test papers of the present invention.

### Example 3

A glass fiber reagent layer having a sample addition section of 5 mm x 10 mm, and a nitrocellulose development layer of 5 mm x 10 mm were adhered in this order on a white polyethylene terephthalate substrate of 5 mm x 65 mm, with overlapped margins of 2 mm each to prepare test papers. The reagent layer had previously had 100 µl/cm² (50 µl/test) of a solution of 10 mg/ml of nicotinamide adenine dinucleotide, 50 units/ml of 3-hydroxybutyrate dehydrogenase, 200 units/ml of diaphorase, 4.5 mg/ml of nitrotetrazolium blue, 10 mg/ml of bovine serum albumin, 10 mg/ml of sucrose, and 4% anti-human red blood cell rabbit antibodies (0.18 mg/ml anti-human red blood cell rabbit antibody IgG fractions) in 40 mM phosphate buffer, pH 8.0 added and was dried under vacuum.
Glass Fiber (Reagent Layer): available from Millipore Corporation (USA) Cat. No. SA3J763H8
Nitrocellulose (Development Layer): available from Millipore Corporation (USA) Cat. No. STHF04000

### Test Example 5

Added to the test papers (Example 3) according to the present invention was 30 µl of whole blood in 12, 114, 545, 1179, 1491, 4765, and 6930 µM concentrations achieved by adding 3-hydroxybutyrate thereto. A reflection rate of 530 nm was measured 2 minutes later using a reflection rate measurement device.

The red blood cells in the whole blood were captured by the glass fiber reagent layer having a sample addition section and the purple color of nitrotetrazolium blue could be identified in the development layer. A calibration curve was prepared with the abscissa representing the 3-hydroxybutyrate concentration in the whole blood and the ordinate representing the reciprocal number of the reflection rate. The results thereof are given in FIG. 9 and Table 5.

**Table 5**

| 3-hydroxybutyrate calibration curve for whole blood | | | | | | | |
|---|---|---|---|---|---|---|---|
| 3-Hydroxybutyrate Concentration in µM | 12 | 114 | 545 | 1179 | 1491 | 4765 | 6930 |
| Reciprocal number of Rate Reflection Rate (%) | 0.0230 | 0.0257 | 0.0466 | 0.0725 | 0.1015 | 0.4065 | 0.5952 |

As apparent from FIG. 9, with the test papers of the present invention, the calibration curve has a good linearity over 0 to 7000 µM for the whole blood samples. The correlation coefficient is as good as R² = 0.9929. The quantitative measurement can be conducted over the wide concentration range.

### Example 4

A glass fiber reagent layer having a sample addition section of 5 mm x 8 mm, a plasma separation membrane of 5 mm x 12 mm, a nitrocellulose development layer of 5 mm x 9 mm, and a filter paper specimen-absorbing layer of 5 mm x 10 mm were adhered in this order on a transparent polyethylene terephthalate substrate of 5 mm x 65 mm, with overlapped margins of 6 mm, 2 mm , and 2 mm each. In the preparatin of the test papers, the test paper was covered with a top laminate of white YUPO synthetic paper of 5 x 25 mm with the sample addition section of 5 mm x 4 mm remaining.

The reagent layer had previously had 75 µl/cm² (30 µl/test) of a solution of 20 mg/ml of nicotinamide adenine dinucleotide, 50 units/ml of 3-hydroxybutyrate dehydrogenase, 200 units/ml of diaphorase, 100 units/ml of ascorbate oxidase, 0.05% of Triton X-100, 10 mg/ml of bovine serum albumin, and 2.25 mg/ml of nitrotetrazolium blue in 40 mM phosphate buffer, pH 8.0 added and was dried under vacuum.
Glass Fiber (Reagent Layer): available from Millipore Corporation (USA) GFF Conjugate Pad Cat. No. SA3J763H8
Nitrocellulose (Development Layer): available from Millipore Corporation (USA) Hi-Flow Plus Nitrocellulose Membrane Cat. No. SHF180
Filter Paper (Specimen-Absorbing Layer): available from Whatman(GB)3MM chr Cat. No. 3030 909

### Test Example 6

Added to the test papers (Example 4) according to the present invention was 30 µl of whole blood in 5, 58, 108, 422, 1062, 2084, 3348, and 5610 µM concentrations achieved by adding 3-hydroxybutyrate thereto. A reflection rate of 635 nm was measured 2 minutes later using a reflection rate measurement device. Five measurements were repeated for each concentration. The reflection rate (R) was converted into a K/S value using the Kubelka-Munk equation, i.e., K/S = (1 - R)²/2R.

The red blood cells in the whole blood were captured by the glass fiber reagent layer having a sample addition section and the plasma separation membrane. Development of color could be determined in the development layer, without being affected by the color of the red blood cells. A calibration curve was prepared with the ordinate representing the concentration of 3-hydroxybutyrate in the whole blood and the abscissa representing the K/S value. The results thereof are given in FIG. 10 and Table 6.

**Table 6**

| 3-hydroxybutyrate calibration curve for whole blood | | | | | | | | |
|---|---|---|---|---|---|---|---|---|
| 3-Hydroxybutyrate Concentration in µM | 5 | 58 | 108 | 422 | 1062 | 2084 | 3348 | 5610 |
| Measured Value, Average (N = 5) in µM | 25 | 54 | 85 | 432 | 1058 | 2089 | 3348 | 5616 |
| CV% of Measured Values (N = 5) | 11.6 | 8.1 | 7.4 | 2.7 | 3.3 | 5.8 | 2.6 | 7.7 |

As apparent from FIG. 10, the test papers of the present invention exhibited a good correlation coefficient of R² = 1 in an approximate curve over 0 to 5600 µM even for the whole blood samples. No variation in developed color was found. Five measurements indicated that the CV% of the measured values was within 10% over a wide concentration range of 50 to 5600 µM.

## Claims

1. A test paper comprising; a reagent layer having a sample addition section to which a liquid sample can be added, said reagent layer being impregnated with a reagent that forms a color developing substance based on a substance to be analyzed in the liquid sample; and a development layer that is disposed in contact with said reagent layer, said development layer being adapted to diffuse and develop the color developing substance.

2. A test paper comprising a sample addition layer to which a liquid sample can be added; a reagent layer that is disposed in contact with said sample addition layer, said reagent layer being impregnated with a reagent that forms a color developing substance based on a substance to be analyzed in the liquid sample that is added to said sample addition layer; and a development layer that is disposed in contact with said reagent layer, said development layer being adapted to diffuse and develop the color developing substance.

3. The test paper as claimed in Claim 1 or Claim 2, further comprising a specimen-absorbing layer that is disposed in contact with the development layer.

4. The test paper as claimed in any one of Claims 1 to 3, further comprising a plasma separation membrane that is disposed between the reagent layer and the development layer in contact with said reagent layer and said development layer.

5. The test paper as claimed in any one of Claims 1 to 4, **characterized in that** the development layer is made of a material selected from the group consisting of nitrocellulose, nylon, and poly vinylidene difluoride.

6. The test paper as claimed in any one of Claims 1 to 5, **characterized in that** one or more agglutinating agents are contained in the reagent layer, the sample addition layer, or both of the reagent layer and the sample addition layer, said agglutinating agent having the effect of agglutinating red blood cells and selected from the group consisting of lectin, anti-red blood cell antibodies, and cationic polymers.

7. The test paper as claimed in any one of Claims 1 to 6, **characterized in that** said test paper being adhered on a water-proof substrate.
